# EUROPEAN PATENT APPLICATION

(11) **EP 1 532 952 A1**
(43) Date of publication of application: **25.05.2005**
(21) Application number: 04257152.1
(22) Date of filing: 18.11.2004
(51) Int. Cl.: A61F 9/013, A61F 9/01, A61B 18/12

(54) **Thermokeratoplasty system with a calibrated radio frequency amplifier**

(30) Priority: 19.11.2003 US 718164
(71) Applicant: Refractec, Inc., Irvine, CA 92618 (US)
(72) Inventor: Goth, Paul R., Bremen, ME 04551 (US); Bowers, William J., Highlands Ranch, CO 80130 (US)
(74) Representative: Wombwell, Francis

(57) **Abstract**

A radio frequency amplifier for a system that can correct the patient's vision by reshaping the cornea by applying a proper amount of RF energy. The system includes an electrode that is connected to the output of the radio frequency amplifier and placed in contact with a cornea. The radio frequency amplifier delivers a RF current to the electrode that flows through and denatures the cornea then returns back to the radio frequency amplifier through a return electrode. The electrode can be placed in a circular pattern about the cornea to correct for a hyperopic condition. To effectively provide for vision correction the radio frequency amplifier should ideally provide power at a desired power curve. The radio frequency amplifier is calibrated to provide an actual power curve that is within +/- 10% of the desired power curve within the operating range of the procedure. This insures that the right amount of power is applied to the cornea during the specified activation time.

## Description

### BACKGROUND OF THE INVENTION

### 1. Field of the Invention

The present invention relates to a thermokeratoplasty system that is used to reshape a cornea.

### 2. Prior Art

Techniques for correcting vision have included reshaping the cornea of the eye. For example, myopic conditions can be corrected by cutting a number of small incisions in the corneal membrane. The incisions allow the corneal membrane to relax and increase the radius of the cornea. The incisions are typically created with either a laser or a precision knife. The procedure for creating incisions to correct myopic defects is commonly referred to as radial keratotomy and is well known in the art.

Radial keratotomy techniques generally make incisions that penetrate approximately 95% of the cornea. Penetrating the cornea to such a depth increases the risk of puncturing the Descemets membrane and the endothelium layer, and creating permanent damage to the eye. Additionally, light entering the cornea at the incision sight is refracted by the incision scar and produces a glaring effect in the visual field. The glare effect of the scar produces impaired night vision for the patient.

The techniques of radial keratotomy are only effective in correcting myopia. Radial keratotomy cannot be used to correct an eye condition such as hyperopia. Additionally, keratotomy has limited use in reducing or correcting an astigmatism. The cornea of a patient with hyperopia is relatively flat (large spherical radius). A flat cornea creates a lens system which does not correctly focus the viewed image onto the retina of the eye. Hyperopia can be corrected by reshaping the eye to decrease the spherical radius of the cornea. It has been found that hyperopia can be corrected by heating and denaturing local regions of the cornea. The denatured tis'sue contracts and changes the shape of the cornea and corrects the optical characteristics of the eye. The procedure of heating the corneal membrane to correct a patient's vision is commonly referred to as thermokeratoplasty.

U.S. Patent No. 4,461,294 issued to Baron; U.S. Patent No. 4,976,709 issued to Sand and PCT Publication WO 90/12618, all disclose thermokeratoplasty techniques which utilize a laser to heat the cornea. The energy of the laser generates localized heat within the corneal stroma through photonic absorption. The heated areas of the stroma then shrink to change the shape of the eye.

Although effective in reshaping the eye, the laser based systems of the Baron, Sand and PCT references are relatively expensive to produce, have a non-uniform thermal conduction profile, are not self limiting, are susceptible to providing too much heat to the eye, may induce astigmatism and produce excessive adjacent tissue damage, and require long term stabilization of the eye. Expensive laser systems increase the cost of the procedure and are economically impractical to gain widespread market acceptance and use.

Additionally, laser thermokeratoplasty techniques non-uniformly shrink the stroma without shrinking the Bowmans layer. Shrinking the stroma without a corresponding shrinkage of the Bowmans layer, creates a mechanical strain in the cornea. The mechanical strain may produce an undesirable reshaping of the cornea and probable regression of the visual acuity correction as the corneal lesion heals. Laser techniques may also perforate Bowmans layer and leave a leucoma within the visual field of the eye.

U.S. Patent Nos. 4,326,529 and 4,381,007 issued to Doss et al, disclose electrodes that are used to heat large areas, of the cornea to correct for myopia. The electrode is located within a sleeve that suspends the electrode tip from the surface of the eye. An isotropic saline solution is irrigated through the electrode and aspirated through a channel formed between the outer surface of the electrode and the inner surface of the sleeve. The saline solution provides an electrically conductive medium between the electrode and the corneal membrane. The current from the electrode heats the outer layers of the cornea. Heating the outer eye tissue causes the cornea to shrink into a new radial shape. The saline solution also functions as a coolant which cools the outer epithelium layer.

The saline solution of the Doss device spreads the current of the electrode over a relatively large area of the cornea. Consequently, thermokeratoplasty techniques using the Doss device are limited to reshaped corneas with relatively large and undesirable denatured areas within the visual axis of the eye. The electrode device of the Doss system is also relatively complex and cumbersome to use.

"A Technique for the Selective Heating of Corneal Stroma" Doss et al., Contact & Intraoccular Lens Medical Jrl., Vol. 6, No. 1, pp. 13-17, Jan-Mar., 1980, discusses a procedure wherein the circulating saline electrode (CSE) of the Doss patent was used to heat a pig cornea. The electrode provided 30 volts r.m.s. for 4 seconds. The results showed that the stroma was heated to 70°C and the Bowman's membrane was heated 45°C, a temperature below the 50-55°C required to shrink the cornea without regression.

"The Need For Prompt Prospective Investigation" McDonnell, Refractive & Corneal Surgery, Vol. 5, Jan./Feb., 1989 discusses the merits of corneal reshaping by thermokeratoplasty techniques. The article discusses a procedure wherein a stromal collagen was heated by radio frequency waves to correct for a keratoconus condition. As the article reports, the patient had an initial profound flattening of the eye followed by significant regression within weeks of the procedure.

"Regression of Effect Following Radial Thermokeratoplasty in Humans" Feldman et al., Refractive and Corneal Surgery, Vol. 5, Sept./Oct., 1989, discusses another thermokeratoplasty technique for correcting hyperopia. Feldman inserted a probe into four different locations of the cornea. The probe was heated to 600°C and was inserted into the cornea for 0.3 seconds. Like the procedure discussed in the McDonnell article, the Feldman technique initially reduced hyperopia, but the patients had a significant regression within 9 months of the procedure.

Refractec, Inc. of Irvine California, the assignee of the present application, has developed a system to correct hyperopia with a thermokeratoplasty probe that is in direct contact with the cornea. The probe includes a tip that is inserted down into the stroma layer of a cornea. Electrical current flows through the eye to denature the collagen tissue within the stroma. The process of inserting the probe tip and applying electrical current can be repeated throughout the cornea. The denatured tissue will change the refractive characteristics of the eye. The procedure is taught by Refractec under the service marks CONDUCTIVE KERATOPLASTY and CK.

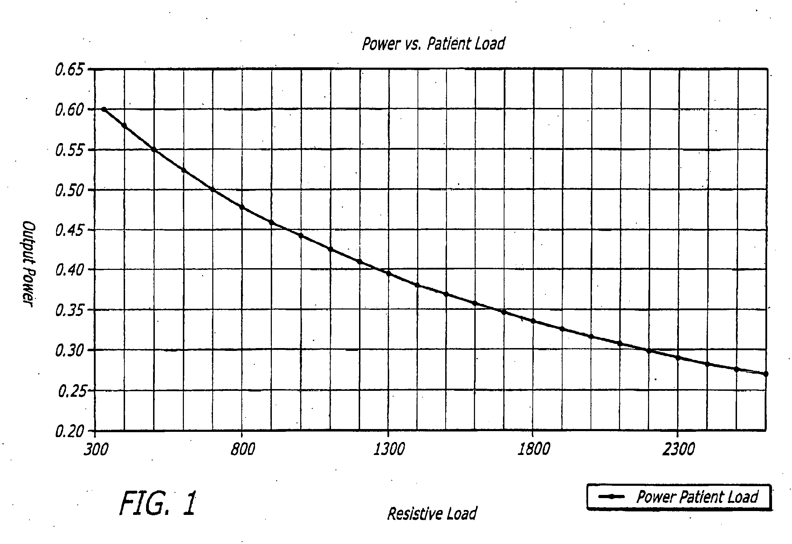

The Refractec system generates a series of damped waveforms in the radio frequency range. Through empirical analysis it has been determined that the technique is most effective if the system delivers a certain power curve to the cornea. Figure 1 shows a desired power curve versus the resistance of the patient. As shown by Fig. 1, the power decreases with an increase in patient impedance.

In accordance with standard electrosurgical calibration procedures, the Refractec generator was calibrated for a desired power at a patient load of 330 ohms. The ohmic operating range for a CK procedure is typically between 600 to 1300 ohms. It was found that calibrating the generator at 330 ohms would produce a power curve that deviated from the ideal curve in the 600 to 1300 ohm operating range of a CK procedure.

### BRIEF SUMMARY OF THE INVENTION

A radio frequency (RF) amplifier that provides electrical power to an electrode placed in contact with a cornea. The cornea can be denatured with a desired power curve to create a desired vision correction. The radio frequency amplifier is calibrated to provide an actual power curve to the cornea within +/- 10% of the desired power curve over an operating range of the tissue impedance.

### BRIEF DESCRIPTION OF THE DRAWINGS

Figure 1 is a graph showing a desired power curve for a CK procedure;
Figure 2 is a perspective view of a thermokeratoplasty electrode system;
Figure 3 is a graph showing a waveform that is provided by a power supply of the system;
Figure 4 is an enlarged view of an electrode inserted into a cornea;
Figure 5 is a top view showing a pattern of denatured areas of the cornea;
Figure 6 is a schematic of a radio frequency electric circuit of the power supply;
Figure 7 is a graph showing a voltage, a current and an impedance at a cornea during a CK procedure;
Figure 8 is a graph showing an actual power curve relative to a desired power curve.

### DETAILED DESCRIPTION

Disclosed is a radio frequency amplifier for a system that can correct the vision of a patient's cornea. The system includes an electrode that is connected to the radio frequency amplifier and placed in contact with a cornea. The radio frequency amplifier delivers a current to the electrode that flows through and denatures the cornea. The electrode can be placed in a circular pattern about the cornea to correct for a hyperopic condition. To effectively provide for vision correction the radio frequency amplifier should ideally provide power at a desired power curve. The radio frequency amplifier is calibrated to provide an actual power curve that is within +/- 10% of the desired power curve within the operating range of the procedure. This insures that the right amount of power is applied to the cornea.

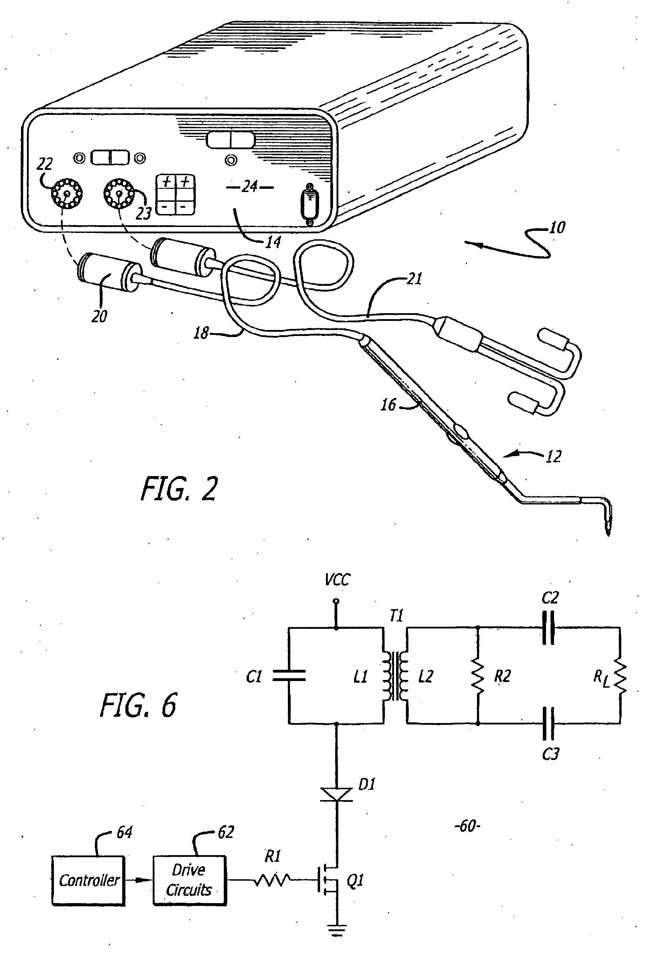

Referring to the drawings more particularly by reference numbers, Figure 2 shows a thermokeratoplasty electrode system 10 of the present invention. The system 10 includes an electrode probe 12 coupled to a power supply unit 14. The power supply unit 14 contains a power supply that can deliver electrical power to the probe 12. The probe 12 has a hand piece 16 and wires 18 that couple the probe electrode to a connector 20 that plugs into a mating receptacle 22 located on the front panel 24 of the generator 14. The hand piece 16 may be constructed from a non-conductive material.

The system 10 also includes a return element 21 that is in contact with the patient to provide a return path for the electrical current provided by the radio frequency amplifier to the probe 12. The return element plugs into a mating receptacle 23 located on the front panel 24 of the generator 14. By way of example, the ground element may be a lid speculum that is used to maintain the patient's eyelids in an open position while providing a return path for the electrical current.

The generator 14 provides a predetermined amount of energy, through a controlled application of power for a predetermined time duration. The generator 14 may have manual controls that allow the user to select treatment parameters such as the power and time duration. The generator 14 can also be constructed to provide an automated operation. The generator 14 may have monitors and feedback systems for measuring tissue impedance, tissue temperature and other parameters, and adjust the output power of the radio frequency amplifier to accomplish the desired results. The unit may also have a display that indicates the number of remaining uses available for the probe 12.

In one embodiment, the power supply provides a constant current source and voltage limiting to prevent arcing. To protect the patient from overvoltage or overpower, the power unit 14 may have an upper voltage limit and/or upper power limit which terminates power to the probe when the output voltage or power of the unit exceeds a predetermined value.

The generator 14 may also contain monitor and alarm circuits which monitors the resistance or impedance of the load and provide an alarm when the resistance/impedance value exceeds and/or falls below predefined limits. The alarm may provide either an audio and/or visual indication to the user that the resistance/impedance value has exceeded the outer predefined limits. Additionally, the unit may contain a ground fault indicator, and/or a tissue temperature monitor. The front panel of the power unit typically contains meters and displays that provide an indication of the power, frequency, etc., of the power delivered to the probe.

The generator 14 may deliver a power output in a frequency range of 300 KHz- 5 MHz. In the preferred embodiment, power is provided to the probe at a frequency in the range of 350 KHz. The generator 14 is designed so that the power supplied to the probe 12 does not exceed 1.2 watts (W). The time duration of each application of power to a particular corneal location is typically between 0.1-1.0 seconds. The unit 14 is preferably set to deliver approximately .75 W of power for 0.75 seconds.

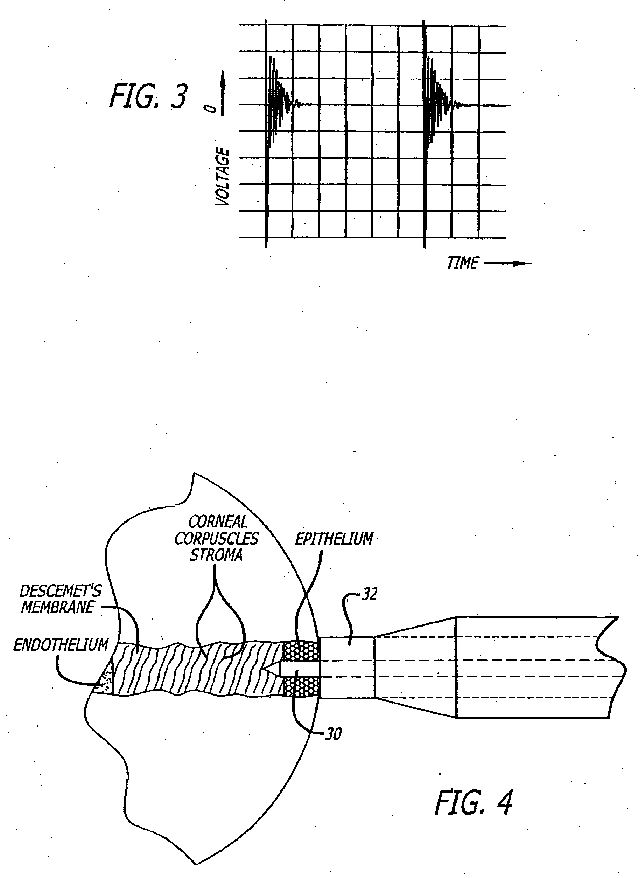

Figure 3 shows a typical voltage waveform that is applied by the generator 14. Each pulse of energy delivered by the generator 14 is a highly damped sinusoidal waveform, typically having a crest factor (peak voltage/RMS voltage) greater than 5:1. Each highly damped sinusoidal waveform is repeated at a repetitive rate. The repetitive rate may range between 4-12 KHz and is preferably set at 7.5 KHz.

As shown in Figure 4, during a procedure, an electrode tip 30 of the handpiece is inserted into a cornea. The length of the tip 30 is typically 300-600 microns, preferably 400 microns, so that the electrode enters the stroma. The electrode may have a stop 32 that limits the penetration of the tip 30. The tip diameter is small to minimize the invasion of the eye.

The radio frequency amplifier provides a current to the cornea through the tip 30. The current denatures the stroma 'to correct the shape of the cornea. Because the tip 30 is inserted into the stroma it has been found that a power no greater than 1.2 watts for a time duration no greater than 1.0 seconds will adequately denature the corneal tissue to provide optical correction of the eye. Inserting the tip 30 into the cornea provides improved repeatability over probes placed into contact with the surface of the cornea, by reducing the variances in the electrical characteristics of the epithelium and the outer surface of the cornea.

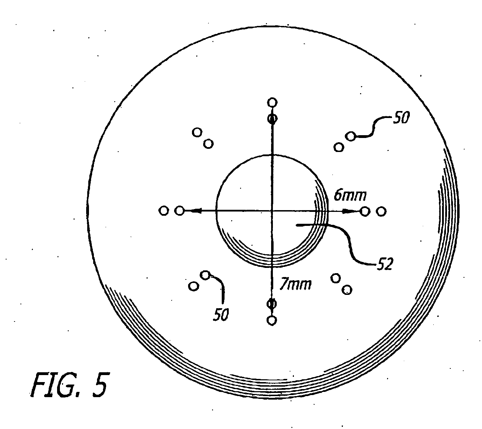

Figure 5 shows a pattern of denatured areas 50 that have been found to correct hyperopic conditions. A circle of 8, 16, or 24 denatured areas 50 are created about the center of the cornea, outside the visual axis portion 52 of the eye. The visual axis has a nominal diameter of approximately 5 millimeters. It has been found that 16 denatured areas provide the most corneal shrinkage and less post-op astigmatism effects from the procedure. The circle of denatured areas typically have a diameter between 6-8 mm, with a preferred diameter of approximately 7 mm. If the first circle does not correct the eye deficiency, the same pattern may be repeated, or another pattern of 8 denatured areas may be created within a circle having a diameter of approximately 6.0-6.5 mm either in line or overlapping. The assignee of the present application provides instructional services to educate those performing such procedures under the service marks CONDUCTIVE KERATOPLASTY and CK.

The exact diameter of the pattern may vary from patient to patient, it being understood that the denatured spots should preferably be formed in the non-visionary portion 52 of the eye. Although a circular pattern is shown, it is to be understood that the denatured areas may be located in any location and in any pattern. In addition to correcting for hyperopia, the present invention may be used to correct astigmatic conditions. For correcting astigmatic conditions, the denatured areas are typically created at the end of the astigmatic flat axis. The present invention may also be used to correct procedures that have overcorrected for a myopic condition.

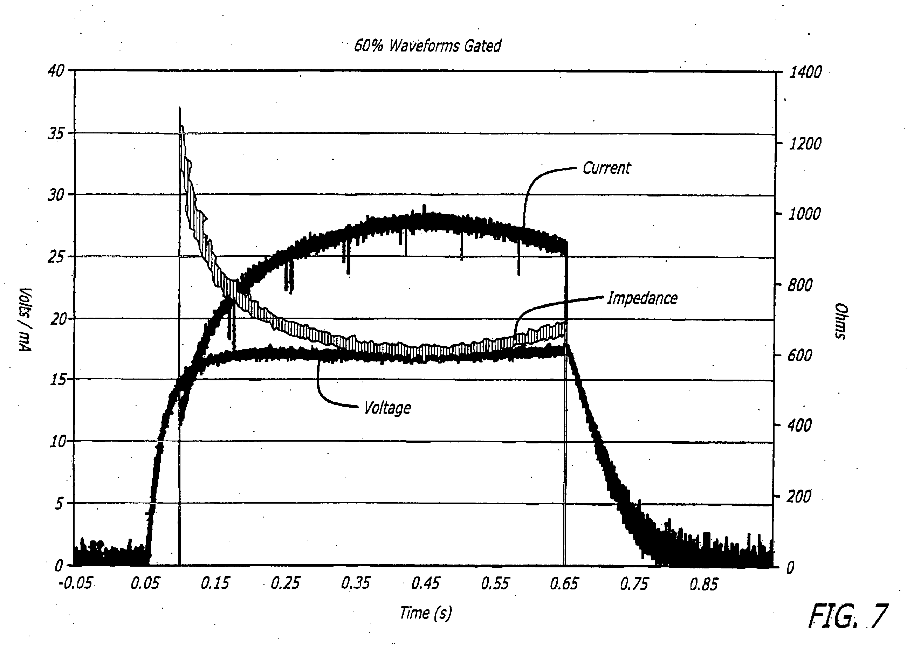

Figure 6 shows a radio frequency ("RF") amplifier 60 of the generator that can apply RF power to a patient's cornea. The patient is represented by resistor R_{L}. The circuit 60 may include a transformer T1 that both stores and discharges energy. The transformer T1 has a primary winding L1 that is connected to a voltage supply line Vcc and to a switch Q1 by diode D1. The primary winding L1 is connected in parallel with capacitor C1.

The switch Q1 may be a MOSFET transistor with a gate coupled to a driver circuit 62 through resistor Rl. The driver circuit 62 may be connected to a controller 64 that can turn the switch on and off. The circuit 60 may further have a pre-load resistor R2 and capacitors C2 and C3 connected to a secondary winding L2 of the transformer T1. The capacitors C2 and C3 filter undesirable low frequency current from flowing into the load R_{L}. The pre-load resistor pulls some of the current from the winding through current division so that a constant power is not provided to the load R_{L}. The ohmic value of the pre-load resistor is selected to obtain the desired power curve.

In operation, the controller 64 turns the switch Q1 on which causes the primary winding L1 to store energy in the magnetic material of T1. The controller 64 turns off the switch Q1, wherein the magnetic material of the transformer T1 discharges its stored energy. The discharge creates a current in the primary loop with the capacitor C1. The frequency of the current is established by the capacitance and inductance values of the capacitor and inductor L1, respectively. The current is also induced onto the secondary winding L2 and applied to the patient load R_{L}. An example of the resultant waveform is shown Fig. 3.

Application of current to a cornea will denature the cornea tissue and cause corresponding change in the ohmic value of the patient load resistance R_{L}. The current that flows through the load will change inversely with a variation in the resistance value of R_{L}. This is shown in Figure 7, which shows the voltage, current and impedance at the patient load R_{L} during the application of power to a cornea. In a typical CK procedure the impedance of the load resistor R_{L} starts at approximately 1300 ohms and falls to approximately 600 ohms before increasing again as shown in Fig. 7, although the impedance may range from 330 to 2600 ohms.

The change in current will also change the power applied to the cornea. This can be seen from the desired power curve shown in Fig. 1. The power decreases with an increase in the patient load R_{L}. It has been found that the desired power curve shown in Fig. 1 provides the most satisfactory results when correcting for hyperopia. The power will ideally increase from approximately .39 watts when the patient load is 1300 ohms to .52 watts when the patient load falls to 600 ohms (see also Fig. 7). A deviation from the desired power curve shown in Fig. 1 may cause a regression in the change of the cornea refractive power.

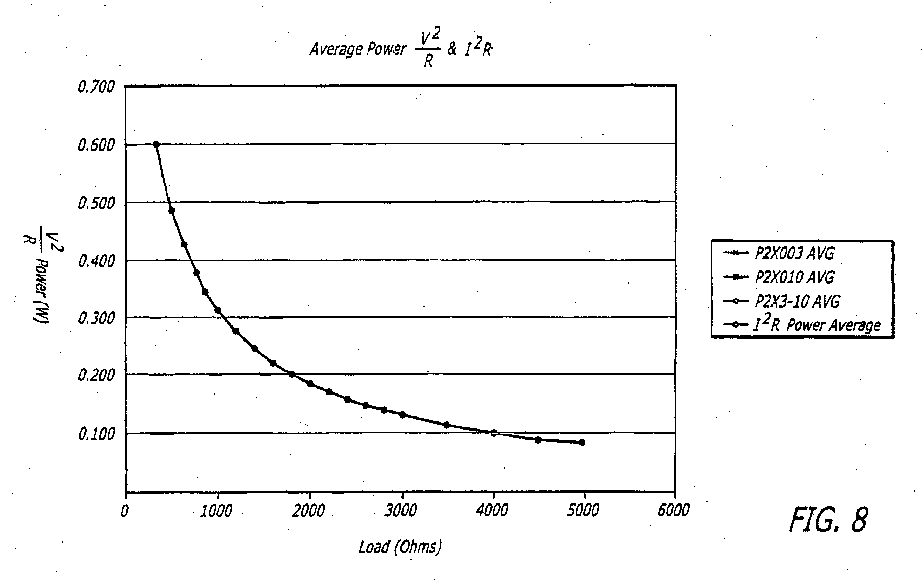

It is desirable to calibrate the electrical circuit 60 so that the power curve actually generated by the circuit 60 and applied to the patient load is within +/- 10% of the desired power curve shown in Fig. 1. By way of example, to calibrate the circuit the pre-load resistor R2 can be set to 1527 ohms. The capacitors C1, C2 and C3 can be set to .00342µ, .033µ and .033µ Farads, respectively. The windings L1 and L2 may have inductance values of 44.73µ and 12.658µ Henrys, respectively. With these values the electrical. circuit has been found to create an ideal power curve as shown in Figure 8. The ideal power curve is within the +/-10% criteria. Meeting this criteria has been found to minimize patient regression after a CK procedure.

While certain exemplary embodiments have been described and shown in the accompanying drawings, it is to be understood that such embodiments are merely illustrative of and not restrictive on the broad invention, and that this invention not be limited to the specific constructions and arrangements shown and described, since various other modifications may occur to those ordinarily skilled in the. art.

## Claims

1. A radio frequency amplifier that provides an electrical power to an electrode placed in contact with a cornea having a tissue impedance, comprising:
a radio frequency electrical circuit calibrated to' provide an actual power curve to the cornea within +/- 10% of a desired power curve over an operating range of the tissue impedance.

2. The radio frequency amplifier of claim 1, wherein said radio frequency electrical circuit includes an transformer, a capacitor, and a pre-load resistor in parallel with a patient load resistance.

3. The radio frequency amplifier of claim 1, wherein the actual power curve varies between .6 to .15 watts.

4. The radio frequency amplifier of claim 3, wherein radio frequency electrical circuit applies power to a load with an impedance that varies between 330 to 2600 ohms.

5. The radio frequency amplifier of claim 1, wherein said radio frequency electrical circuit generates a series of damped waveforms.

6. The radio frequency amplifier of claim 1, wherein the operating range of the actual power curve has a time duration less than 1 second.

7. A radio frequency amplifier that provides an electrical power to an electrode placed in contact with a cornea having a tissue impedance, comprising:
radio frequency circuit means for providing an actual power curve to the cornea within +/- 10% of a desired power curve over an operating range of the tissue impedance.

8. The radio frequency amplifier of claim 7, wherein said radio frequency circuit means includes an transformer, a capacitor, and a pre-load resistor in parallel with patient load resistance.

9. The radio frequency amplifier of claim 7, wherein the actual power curve varies.between .6 to .15 watts.

10. The radio frequency amplifier of claim 9, wherein radio frequency circuit means applies power to a load with an impedance that varies between 330 to 2600 ohms.

11. The radio frequency amplifier of claim 7, wherein said radio frequency circuit means generates a series of damped waveforms.

12. The power supply of claim 7, wherein the operating range of the actual power curve has a time duration less than 1 second.

13. A medical system that can denature a cornea having a tissue impedance, comprising:
a radio frequency electrical circuit calibrated to provide an actual power curve to the cornea within +/- 10% of a desired power curve over an operating range of the tissue impedance;
an electrode coupled to said radio frequency electrical circuit and which is placed into contact with the cornea; and,
a ground element coupled to said radio frequency electrical circuit.

14. The system of claim 13, wherein said radio frequency electrical circuit includes a transformer, a capacitor, and a pre-load resistor in parallel with patient load resistance.

15. The system of claim 13, wherein the actual power curve varies between .6 to .15 watts.

16. The system of claim 15, wherein said radio frequency electrical circuit applies power to a load with an impedance that varies between 330 to 2600 ohms.

17. The system of claim 13, wherein said radio frequency electrical circuit generates a series of damped waveforms.

18. The system of claim 13, wherein the operating range of the actual power curve has a time duration less than 1 second.

19. A medical system that can denature a cornea having a tissue impedance, comprising:
an electrode that is placed into contact with the cornea;
radio frequency circuit means for providing an actual power curve to said electrode and the cornea within +/- 10% of a desired power curve over an operating range of the tissue impedance; and,
a ground element coupled to said radio frequency circuit means.

20. The system of claim 19, wherein said radio frequency circuit means includes a transformer, a capacitor, and a pre-load resistor in parallel with patient load resistance.

21. The system of claim 19, wherein the actual power curve varies between .6 to 15 watts.

22. The system of claim 21, wherein radio frequency circuit means applies power to a load with an impedance. that varies between 330 to 2600 ohms.

23. The system of claim 19, wherein said radio frequency circuit means generates a series of damped waveforms.

24. The power supply of claim 19, wherein the operating range of the actual power curve has a time duration less than 1 second.

25. A method for correcting a vision of a cornea having a tissue impedance, comprising:
applying power to the cornea with a power curve that is within +/- 10% of a desired power curve over an operating range of the tissue impedance.

26. The method of claim 25, wherein the power is applied in a circular pattern about the cornea.

27. The method of claim 26, wherein the circular pattern has a diameter between 6 to 8 millimeters.

28. The method of claim 25, wherein a tip of the electrode is inserted into the cornea.

29. The method of claim 25, wherein the applied power varies between .6 to .15 watts.

30. The method of claim 25, wherein the power is applied to a load with an impedance that varies between 330 to 2600 ohms.
